# EUROPEAN PATENT SPECIFICATION

(11) **EP 4 148 144 B1**
(45) Date of publication and mention of the grant of the patent: **29.05.2024**
(21) Application number: 22157315.7
(22) Date of filing: 17.02.2022
(51) Int. Cl.: C12Q 1/6816

(54) **COMPOSITION FOR IDENTIFYING ERYTHROPOIETIN GENE DOPING AND USE THEREOF**
ZUSAMMENSETZUNG ZUR IDENTIFIZIERUNG VON ERYTHROPOIETIN-GEN-DOTIERUNG UND DEREN VERWENDUNG
COMPOSITION POUR IDENTIFIER LE DOPAGE GÉNÉTIQUE DE L'ÉRYTHROPOÏÉTINE ET SON UTILISATION

(30) Priority: 13.09.2021 KR 20210121631
(43) Date of publication of application: 15.03.2023
(73) Proprietor: Korea Institute of Science and Technology, Seoul 02792 (KR)
(72) Inventor: SUNG, Changmin, 02792 Seoul (KR); SON, Junghyun, 02792 Seoul (KR); YI, Joonyeop, 02792 Seoul (KR)
(74) Representative: advotec.

(56) References cited:
- CN-A- 111 575 351
- CN-A- 112 063 604
- YI JOON-YEOP ET AL: "New application of the CRISPR-Cas9 system for site-specific exogenous gene doping analysis", DRUG TESTING AND ANALYSIS, vol. 13, no. 4, 24 January 2021 (2021-01-24), pages 871-875, XP055947505, GB ISSN: 1942-7603, DOI: 10.1002/dta.2980
- BAOUTINA A ET AL: "Gene doping detection: evaluation of approach for direct detection of gene transfer using erythropoietin as a model system", GENE THERAPY, NATURE PUBLISHING GROUP, LONDON, GB, vol. 17, no. 8, 13 May 2010 (2010-05-13), pages 1022-1032, XP037771432, ISSN: 0969-7128, DOI: 10.1038/GT.2010.49 [retrieved on 2010-05-13]
- PASSREITER ALINA ET AL: "First Steps toward Uncovering Gene Doping with CRISPR/Cas by Identifying SpCas9 in Plasma via HPLC-HRMS/MS", ANALYTICAL CHEMISTRY, vol. 92, no. 24, 25 November 2020 (2020-11-25), pages 16322-16328, XP055947680, US ISSN: 0003-2700, DOI: 10.1021/acs.analchem.0c04445

## Description

### BACKGROUND OF THE INVENTION

### 1. Field of the Invention

The present invention relates to a composition for identifying erythropoietin gene doping, a kit for identifying erythropoietin gene doping, an information provision system for identifying erythropoietin gene doping, a method for providing information necessary for identifying erythropoietin gene doping, and a method for identifying erythropoietin gene doping.

### 2. Description of the Related Art

Since 2001, the World Anti-Doping Agency (WADA) has funded more than $80 million to develop new analytical techniques for prohibited doping substances and doping methods and improving the ability of the analytical techniques to detect doping agents, which will help researchers around the world make great advances in anti-doping science. The WADA's 2003 Prohibited List included the following descriptions regarding the use of genes for athletic performance enhancement and non-therapeutic use of genetic elements and cells using gene manipulation: "The use of nucleic acids or nucleic acid analogues that may alter genome sequences and/or alter gene expression by any mechanism. This includes but is not limited to gene editing, gene silencing and gene transfer technologies. The use of normal or genetically modified cells".

Gene doping is a concept that stems from gene therapy. Gene therapy refers to a technique that modifies genetic information in patient's specific cells to treat disease. Gene therapy is conducted in such a way that a virus or DNA carrying genetic information for modification is appropriately introduced into a patient to transform cells of interest. Here, modification of the genetic information for performance enhancement rather than for disease treatment is considered gene doping. Erythropoietin is a hormone involved in the production of red blood cells in our body. An increased red blood cell content increases the amount of oxygen supplied to muscles, leading to increased endurance. Doping is based on this effect and has been accomplished by erythropoietin injection. The manipulation of a gene that governs this hormone enables our body to produce more red blood cells on its own. The scary thing about this type of gene doping is that its detection is very difficult because our body performs such functions on its own without the introduction of foreign substances. According to statistics provided by the Journal of Gene Medicine, the number of people who had undergone gene therapy worldwide by 2015 exceeded 2300 and no case of gene doping has been reported to date.

On the other hand, the World Anti-Doping Agency (WADA) issued guidelines for gene doping analysis for the first time in January 2021. This is the first overview of gene doping detection in 18 years after the prohibition of gene doping in 2003. The guidelines suggest detection methods based on real-time polymerase chain reaction (RT-PCR). RT-PCR is a basic technique for amplifying the number of genes and recording the amplified signals. This technique is the key to accurately amplifying target genes. However, once non-target molecules are copied, PCR is very vulnerable to signal contamination due to its exponential amplification nature.

Non-Patent Document 1 (see below) discloses a concept of gene doping analysis using the clustered regularly interspaced short palindromic repeats-CRISPR associated protein 9 (CRISPR-Cas9) system. With overexpressed Cas9 proteins and commercially synthesized single guide RNA (sgRNA), the general protocol of the *in vitro* Cas9 nuclease was modified and optimized to use single or double cleavage assays for antigene doping analysis to detect specific sizes of human erythropoietin (hEPO) cDNA fragments. The *in vitro* Cas9 double cleavage assay used exon-exon specific sgRNA pairs and the results were analyzed using DNA electrophoresis. The Cas9 enzyme was expressed in *E. coli* and purified using a His-tag.

CN 11 2 063 604 A discloses the use of two functionalized dCas9 modified proteins and their application in detecting nucleic acid. The dCas9 protein is a mutant of the Cas9 protein, that is, the RuvC1 and HNH nuclease active regions of the Cas9 endonuclease are mutated at the same time, resulting in the disappearance of the endonuclease activity of the Cas9 protein, and only the ability to guide the sgRNA into the genome is retained. Biotin and alkaline phosphatase were used to label dCas9 protein to obtain functionalized dCas9 modified protein; then, on this basis, solid-phase magnetic beads and CRISPR-guided "sandwich recognition" were used to construct a chemistry for nucleic acid detection. The sandwich recognition improves the specificity of the detection system, and provides an effective means for further reducing the false positive/false negative results of nucleic acid detection. The method uses the alkaline phosphatase coupled to dCas9 to generate a luminescent signal.

CN 11 1 575 351 A discloses a method for the detection of human papilloma virus (HPV) DNA using dCas9 coupled to magnetic beads and to streptavidin-bound HRP. The coupling is achieved through capture sequence on the sgRNA of one of the dCas9-sgRNAs to capture the dCas9-sgRNA-DNA on magnetic beads and capture sequence on the sgRNA of one of the dCas9-sgRNAs to capture a colorimetric marker to dCas9-sgRNA-DNA. Wherein, the pair of dCas9-sgRNA refers to two dCas9-sgRNA complexes, namely dCas9-sgRNAa and dCas9-sgRNAb; wherein sgRNAa and sgRNAb have different 5'-end target DNA binding sequences and 3'-end target DNA binding sequences, respectively.

Non-Patent Document 3 (see below) discloses a method to detect gene doping using erythropoietin (EPO) as a model gene and a simple in vitro system. It discloses a real-time PCR assays that target sequences within the transgene complementary DNA corresponding to exon/exon junctions.

Non-Patent Document 4 (see below) discloses a method for gene doping by identification of the exogenous protein Cas9 from the bacterium *Streptococcus pyogenes* (SpCas9) in plasma samples by means of a bottom-up analytical approach via immunoaffinity purification, tryptic digestion, and subsequent detection by HPLC-HRMS/MS.

Under these circumstances, the present inventors have endeavored to develop a composition or method for identifying gene doping based on the CRISPR/dCas9 system that can directly detect synthetic human erythropoietin (hEPO) gene sequences.

### Prior Art Documents

CN 11 2 063 604 A and CN 11 1 575 351 A.

### Non-Patent Documents

(Non-Patent Document 1) Yi, J-Y, Kim, M, Min, H, et al. New application of the CRISPR-Cas9 system for site-specific exogenous gene doping analysis. Drug Test Anal. 2021; 13: 871-875. https://doi.org/10.1002/dta.2980,
(Non-Patent Document 2) Sternberg SH, Redding S, Jinek M, Greene EC, Doudna JA. DNA interrogation by the CRISPR RNA-guided endonuclease Cas9. Nature. 2014; 507(7490): 62-67,
(Non-Patent Document 3) Baoutina A. et al. Gene doping detection: evaluation of approach for direct detection of gene transfer using erythropoietin as a model system. GENE THERAPY, NATURE PUBLISHING GROUP, LONDON, GB, vol. 1 7, no. 8, 13 May 2010, pages 1022-1032,
(Non-Patent Document 4) Passreiter A., et al. First Steps toward Uncovering Gene Doping with CRISPR/Cas by Identifying SpCas9 in Plasma via HPLC-HRMS/MS. ANALYTICAL CHEMISTRY, vol. 92, no. 24, 25 November 2020, pages 16322-16328.

### SUMMARY OF THE INVENTION

One object of the present invention is to provide a composition for identifying erythropoietin gene doping, including: two sgRNAs specifically binding to an exogenous human erythropoietin gene; and a dCas9 having the amino acid sequence set forth in SEQ ID NO: 5 and the dCas9 contains an affinity tag; and a dCas9 having an amino acid sequence set forth in any one of SEQ ID NOs: 6 to 8 and the dCas9 is biotinylated, and the biotin is labeled with a marker; and an expression substrate that exhibits at least one variation selected from optical, electrical, and chemical variations.

A further object of the present invention is to provide a kit for identifying erythropoietin gene doping, including: two sgRNAs specifically binding to an exogenous human erythropoietin gene; and a dCas9 labeled with an affinity tag; a biotinylated dCas9; a marker binding with the biotin; and an expression substrate that exhibits at least one variation selected from optical, electrical, and chemical variations.

Another object of the present invention is to provide an information provision system for identifying erythropoietin gene doping, including: two sgRNAs specifically binding to an exogenous human erythropoietin gene; and a dCas9 labeled with an affinity tag, a dCas9 labeled with a marker; and an expression preparation unit providing an expression substrate that exhibits at least one variation selected from optical, electrical, and chemical variations.

Still another object of the present invention is to provide a method for identifying erythropoietin gene doping by using two sgRNAs specifically binding to an exogenous human erythropoietin gene; and a dCas9 containing an affinity tag; and a biotinylated dCas9.

One aspect of the present invention provides a composition for identifying erythropoietin gene doping, including: two single guide RNAs (sgRNAs) having different nucleotide sequence set forth in any one of SEQ ID NOs: 1 to 4; and a dead Cas9 or nuclease-inactive Cas9 (dCas9) having the amino acid sequence set forth in SEQ ID NO: 5 and the dCas9 contains an affinity tag; and a dCas9 having an amino acid sequence set forth in any one of SEQ ID NOs: 6 to 8 and the dCas9 is biotinylated, and the biotin is labeled with a marker; and an expression substrate that exhibits at least one variation selected from optical, electrical, and chemical variations.

According to one embodiment of the present invention, the composition may include single guide RNA 2 (sgRNA2) having the nucleotide sequence set forth in SEQ ID NO: 2 and sgRNA3 having the nucleotide sequence set forth in SEQ ID NO: 3.

According to one embodiment of the present invention, the composition may include single guide RNA 2 (sgRNA2) having the nucleotide sequence set forth in SEQ ID NO: 2 and sgRNA1 having the nucleotide sequence set forth in SEQ ID NO: 1.

The composition includes a dCas9 having the amino acid sequence set forth in SEQ ID NO: 5 and the dCas9 may contain an affinity tag.

According to one embodiment of the present invention, the affinity tag may be selected from His tags, FLAG tags, S tags, glutathione S-transferase (GST) tags, maltose binding protein (MBP) tags, chitin binding protein (CMP) tags, Avi tags, calmodulin tags, polyglutamate tags, E tags, HA tags, myc tags, SBP tags, Softag 1, Softag 3, Strap tags, TC tags, Xpress tags, biotin carboxyl carrier protein (BCCP) tags, and green fluorescent protein (GFP) tags.

The composition includes a dCas9 having an amino acid sequence set forth in any one of SEQ ID NOs: 6 to 8, the dCas9 may be biotinylated, and the biotin may be labeled with a marker.

According to one embodiment of the present invention, the marker may be selected from streptavidin-bound horseradish peroxidase (HRP), alkaline phosphatase (AP), β-galactosidase, luciferase, acetylcholinesterase, urease, catalase, asparginase, ribonuclease, malate dehydrogenase, staphylococcal nuclease, triose phosphate isomerase, glucose oxidase, cytochrome P450, and peroxidase compounds.

The expression substrate that exhibits at least one variation selected from optical, electrical, and chemical variations due to the catalytic action of the marker is a chromogenic material, a luminescent material, a fluorescent material or a combination thereof.

The present invention also provides a kit for identifying erythropoietin gene doping, including: two sgRNAs having different nucleotide sequences set forth in any two of SEQ ID NOs: 1 to 4; a dCas9 labeled with an affinity tag; a biotinylated dCas9; a marker binding with the biotin; and an expression substrate that exhibits at least one variation selected from optical, electrical, and chemical variations due to the catalytic action of the marker and includes a chromogenic material, a luminescent material, a fluorescent material or a combination thereof.

The present invention also provides an information provision system for identifying erythropoietin gene doping, including: a targeting unit including a biological sample isolated from an individual suspected of gene doping, a dCas9 labeled with an affinity tag, a dCas9 labeled with a marker, and two sgRNAs having different nucleotide sequences set forth in any two of SEQ ID NOs: 1 to 4; an expression preparation unit providing an expression substrate that exhibits at least one variation selected from optical, electrical, and chemical variations due to the catalytic action of the marker bound to the dCas9 and includes a chromogenic material, a luminescent material, a fluorescent material or a combination thereof; and an expression measurement unit determining whether the individual has gene doped or not based on the variation exhibited by the expression substrate.

The present invention also provides a method for identifying erythropoietin gene doping, including (1) reacting a dCas9 containing an affinity tag with a sgRNA having a nucleotide sequence set forth in any one of SEQ ID NOs: 1 to 4 to form a complex, (2) immobilizing the complex onto magnetic beads via the affinity tag binding to the magnetic beads, (3) reacting the complex immobilized onto the magnetic beads with a biological sample isolated from an individual suspected of gene doping, (4) reacting the reaction product with a sgRNA having a nucleotide sequence set forth in any one of the other SEQ ID NOs: 1 to 4 and a biotinylated dCas9, (5) reacting the reaction product with a marker capable of binding with the biotin, (6) reacting the reaction product with an expression substrate that exhibits at least one variation selected from optical, electrical, and chemical variations due to the catalytic action of the marker, and (7) determining whether the individual has gene doped or not based on the variation exhibited by the expression substrate.

The composition for identifying erythropoietin gene doping, the kit for identifying doping, and the information provision system for identifying doping according to the present invention can rapidly identify erythropoietin gene doping with very high sensitivity and accuracy without the need to isolate the gene or perform PCR. In addition, the method for identifying erythropoietin gene doping according to the present invention is very useful due to its ability to rapidly identify erythropoietin gene doping with very high sensitivity and accuracy.

### BRIEF DESCRIPTION OF THE DRAWINGS

These and/or other aspects and advantages of the invention will become apparent and more readily appreciated from the following description of the embodiments, taken in conjunction with the accompanying drawings of which:

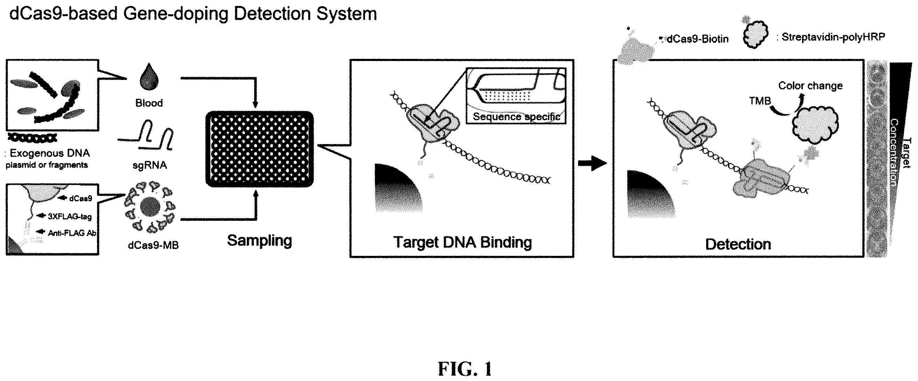

Fig. 1 schematically shows a dCas9-based gene-doping detection system according to one embodiment of the present invention;
Fig. 2 shows the results of SDS-PAGE for dCas9H (SEQ ID NO: 6) and dCas9F (SEQ ID NO: 5) proteins expressed and purified in *E. coli* in Example 1;
Fig. 3 shows the construction of an exogenous human erythropoietin gene and the locations of the gene targeted by sgRNA1 (SEQ ID NO: 1), sgRNA2 (SEQ ID NO: 2), sgRNA3 (SEQ ID NO: 3), and sgRNA4 (SEQ ID NO: 4);
Fig. 4 shows the results of native-PAGE in Example 2, demonstrating that a dCas9 and sgRNAs were attached to different locations of an exogenous human erythropoietin gene;
Fig. 5 shows the results of native-PAGE in Example 2, demonstrating that a dCas9 and sgRNAs were attached to different locations of an exogenous human erythropoietin gene for different reaction times;
Fig. 6 shows optimal biotinylation conditions of a dCas9 determined by observing signals from streptavidin-polyHRP under different biotinylation conditions of the dCas9 depending on sgRNA combinations;
Fig. 7 shows the abilities of C80L (SEQ ID NO: 7) and C574E (SEQ ID NO: 8) as dCas9s to detect genes when the dCas9s were operated;
Fig. 8 compares the operational performances of sensors using C80L mutant and sgRNA combinations to find an optimal sgRNA combination.
Fig. 9 shows the ability of a gene detection sensor according to the present invention to detect an exogenous human erythropoietin gene from an endogenous erythropoietin gene in whole blood when the sensor was operated and the limit of detection of the sensor.

### DETAILED DESCRIPTION OF THE INVENTION

The present invention will now be described in detail.

Human erythropoietin (hEPO) is a glycoprotein hormone involved in the production of red blood cells. The use of human erythropoietin is restricted to athletes and the use of human erythropoietin gene is also banned in athletes. Actions such as the use of genes for athletic performance enhancement and non-therapeutic use of genetic elements and cells using gene manipulation are collectively called gene doping.

First, the use of exogenous genes is assumed to be the most common gene doping method. Exogenous genes consist of exons only ([exon]-[exon]-), unlike endogenous genes having exon and intron sequences such as [intron]-[exon]-[intron]-. Thus, the present inventors have attempted to design sgRNAs that specifically bind to exon-exon junctions, which appears only in exogenous gene sequences, in order to detect exogenous genes in potential doping samples.

Specifically, the present inventors have designed a sgRNA that specifically binds to the sequences of four exon-exon junctions between the sequences of five exons constituting an exogenous human erythropoietin gene and have found that when a dCas9 protein reacts with the sgRNA, the resulting composition can specifically bind to the exogenous human erythropoietin gene. Thereafter, the present inventors have succeeded in isolating the exogenous human erythropoietin by continuously reacting with a sgRNA binding to another location of the gene and a dCas9 protein-HRP complex and in detecting final signals.

In one aspect, the present invention provides a composition for identifying erythropoietin gene doping, including: two single guide RNAs (sgRNAs) having different nucleotide sequence set forth in any one of SEQ ID NOs: 1 to 4; and a dead Cas9 or nuclease-inactive Cas9 (dCas9) having the amino acid sequence set forth in SEQ ID NO: 5 and the dCas9 contains an affinity tag; and a dCas9 having an amino acid sequence set forth in any one of SEQ ID NOs: 6 to 8 and the dCas9 is biotinylated, and the biotin is labeled with a marker; and an expression substrate that exhibits at least one variation selected from optical, electrical, and chemical variations.

The composition of the present invention can detect an exogenous human erythropoietin gene in a biological sample isolated from a subject suspected of erythropoietin (EPO) gene doping to rapidly identify with high accuracy and sensitivity whether or not the subject has doped with the exogenous human erythropoietin gene.

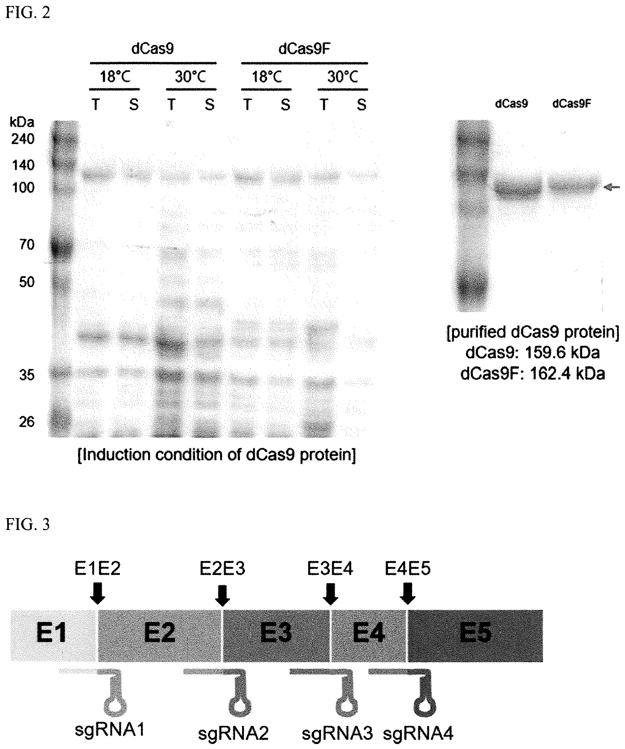

The exogenous human erythropoietin gene consists of five exons and four exon-exon junctions (exon1-exon2, exon2-exon3, exon3-exon4, and exon4-exon5) (see Fig. 3).

As shown in Fig. 3, the sgRNA1 includes a sequence that specifically binds to the exon1-exon2 junction of the exogenous human erythropoietin gene, the sgRNA2 includes a sequence that specifically binds to the exon2-exon3 junction of the exogenous human erythropoietin gene, the sgRNA3 includes a sequence that specifically binds to the exon3-exon4 junction of the exogenous human erythropoietin gene, and the sgRNA4 includes a sequence that specifically binds to the exon4-exon5 junction of the exogenous human erythropoietin gene.

The sgRNA present in the composition for identifying gene doping according to the present invention may be selected from the sgRNA1 to the sgRNA4. According to the invention, the sgRNA is a combination of two of the sgRNA1 to the sgRNA4. More preferably a combination of the sgRNA2 having the nucleotide sequence set forth in SEQ ID NO: 2 and the sgRNA1 having the nucleotide sequence set forth in SEQ ID NO: 1. The sgRNA may be complexed with the dCas9 such that the dCas9 is attached to the target gene.

The composition includes a dCas9 having the amino acid sequence set forth in SEQ ID NO: 5 and the dCas9 contains an affinity tag.

The affinity tag may be selected from His tags, FLAG tags, S tags, glutathione S-transferase (GST) tags, maltose binding protein (MBP) tags, chitin binding protein (CMP) tags, Avi tags, calmodulin tags, polyglutamate tags, E tags, HA tags, myc tags, SBP tags, Softag 1, Softag 3, Strap tags, TC tags, Xpress tags, biotin carboxyl carrier protein (BCCP) tags, and green fluorescent protein (GFP) tags.

The composition includes a dCas9 having an amino acid sequence set forth in any one of SEQ ID NOs: 6 to 8, the dCas9 may be biotinylated, and the biotin may be labeled with a marker.

The marker may be selected from streptavidin-bound horseradish peroxidase (HRP), alkaline phosphatase (AP), β-galactosidase, luciferase, acetylcholinesterase, urease, catalase, asparginase, ribonuclease, malate dehydrogenase, staphylococcal nuclease, triose phosphate isomerase, glucose oxidase, cytochrome P450, and peroxidase compounds.

The composition further includes an expression substrate that exhibits at least one variation selected from optical, electrical, and chemical variations due to the catalytic action of the marker and includes a chromogenic material, a luminescent material, a fluorescent material or a combination thereof.

The concentration of each of the sgRNA selected from the sgRNA1 to the sgRNA4 and the dCas9 in the composition of the present invention is 0.01 to 10 µM, preferably 0.1 to 5 µM, more preferably 0.5 to 4 µM, more preferably 0.5 to 2 µM.

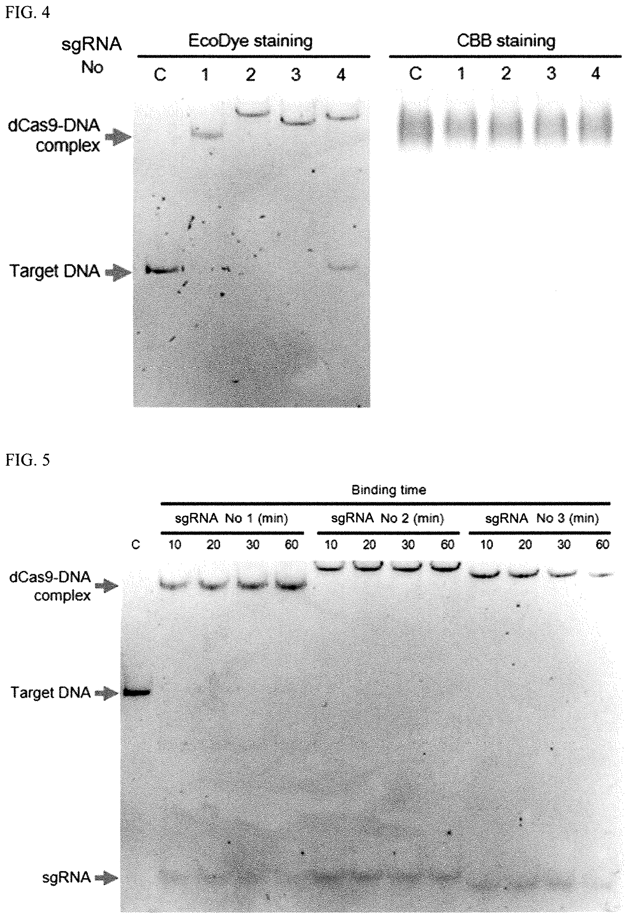

The concentration of each of the sgRNA selected from the sgRNA1 to the sgRNA4 and the dCas9 is preferably at least about 10 times that of the substrate (target gene). The dCas9 is a single turnover enzyme and needs to be used in excess relative to the substrate. Accordingly, it is preferable that the concentration of the sgRNA-dCas9 complex is excessively higher than that of the substrate. Specifically, in the native-PAGE experiments shown in Figs. 4 and 5 (Example 2), the substrate was used at a concentration of 200 nM, which was smaller than that (2 µM) of the dCas9-sgRNA complex (ribonucleoprotein (RNP)).

The dCas9 may be complexed with the sgRNA selected from the sgRNA1 to the sgRNA4 in a molar ratio of 1: 0.5-1.5, preferably 1: 0.8-1.2, more preferably 1:1.

In a further aspect, the present invention provides a kit for identifying erythropoietin gene doping, including: two sgRNAs having different nucleotide sequences set forth in any two of SEQ ID NOs: 1 to 4; a dCas9 labeled with an affinity tag; a biotinylated dCas9; a marker binding with the biotin; and an expression substrate that exhibits at least one variation selected from optical, electrical, and chemical variations due to the catalytic action of the marker and includes a chromogenic material, a luminescent material, a fluorescent material or a combination thereof.

In another aspect, the present invention provides an information provision system including a targeting unit, an expression preparation unit, and an expression measurement unit. The information provision system may be an automatic device or an automated diagnostic system. A description regarding a subject who performs the diagnosis will be omitted for convenience of explanation. The components for performing a plurality of operations are separated. However, this is merely a preferred embodiment for achieving the object of the present invention and it is understood that some of the components may be removed or one or more other components may be added if needed.

The targeting unit includes a biological sample isolated from a subject suspected of gene doping.

The expression preparation unit provides an expression substrate that exhibits at least one variation selected from optical, electrical, and chemical variations due to the catalytic action of a marker bound to a dCas9 and includes a chromogenic material, a luminescent material, a fluorescent material or a combination thereof. The expression substrate may undergo at least one reaction selected from chromogenic reactions, luminescence reactions, and fluorescence reactions due to the catalytic action of the marker.

In one embodiment, the marker may be alkaline phosphatase (AP). In this embodiment, the expression substrate may be a chromogenic material selected from bromo-chloro-indolyl phosphate (BCIP), nitroblue tetrazolium (NBT), naphthol-ASB1-phosphate, and enhanced chemifluorescence (ECF). In an alternative embodiment, the marker may be horseradish peroxidase. In this embodiment, the expression substrate may be selected from chloronaphthol, aminoethylcarbazole, diaminobenzidine, D-luciferin, bis-N-methylacridinium nitrate (lucigenin), resorufin benzyl ether, luminol, Amplex Red reagent (10-acetyl-3,7-dihydroxyphenoxazine), p-phenylenediamine-HCl and pyrocatechol (HYR), tetramethylbenzidine (TMB), 2,2'-azine-di(3-ethylbenzthiazolinesulfonate) (ABTS), O-phenylenediamine (OPD), naphthol/pyronine, glucose oxidase, nitroblue tetrazolium (t-NBT), and phenazine methosulfate (m-PMS). The above-described materials are provided for illustrative purposes only and are not particularly limited as long as they exhibit an optical, electrical or chemical variation.

The expression measurement unit measures light energy from the luminescence, fluorescence reaction or chromogenic reaction of the expression substrate, uses an optical device such as a camera capable of measuring the reaction to identify whether or not the subject has doped with an exogenous human erythropoietin gene, or measures a change in electrical signal such as voltage or current from the expression substrate to identify whether or not the subject has doped with an exogenous human erythropoietin gene.

The information provision system of the present invention may further include a quantitative analysis unit that can measure and quantify signals corresponding to the optical, electrical or chemical variation.

In yet another aspect, the present invention provides a method for identifying erythropoietin gene doping, including (1) reacting a dCas9 containing an affinity tag with a sgRNA having a nucleotide sequence set forth in any one of SEQ ID NOs: 1 to 4 to form a complex, (2) immobilizing the complex onto magnetic beads via the affinity tag binding to the magnetic beads, (3) reacting the complex immobilized onto the magnetic beads with a biological sample isolated from an individual suspected of gene doping, (4) reacting the reaction product with a sgRNA having a nucleotide sequence set forth in any one of the other SEQ ID NOs: 1 to 4 and a biotinylated dCas9, (5) reacting the reaction product with a marker capable of binding with the biotin, (6) reacting the reaction product with an expression substrate that exhibits at least one variation selected from optical, electrical, and chemical variations due to the catalytic action of the marker, and (7) determining whether the individual has gene doped or not based on the variation exhibited by the expression substrate.

The for identifying erythropoietin gene doping according to the present invention directly detects the target gene without amplification, unlike real time-PCR (RT-PCR) as a conventional technique for gene analysis, and have several advantages as follows. First, since the method continuously uses different types of sgRNAs for cross-validation analysis of the target gene sequence, there is a greatly reduced risk of false-positive results caused by non-specific binding of primers during RT-PCR. Second, the method is carried out in a simple manner for a time as short as about 30 to 60 minutes from the sample preparation to the final signal detection and enable both qualitative and quantitative analysis at one time.

Specifically, the method is a sandwich assay. More specifically, according to the methods of the present invention, a sgRNA X is complexed with a dCas9 containing an affinity tag, a sgRNA Y is complexed with a biotinylated dCas9 (dCas9-biotin) to form another complex, and these complexes are independently used for the assays.

Preferred embodiment of the present invention are as follows:
1) the sgRNA X is complexed with the dCas9 containing an affinity tag in a molar ratio of about 1:0.5-1.5, preferably 1:1. The complex is then immobilized onto magnetic beads (MBs) attached with an antibody against the affinity tag;
2) when a sample is added to the dCas9 complex immobilized onto the MBs, the complex binds to the location of the target gene sequence complementary to the sgRNA X sequence. After binding to the target gene, the immobilized complex and the target gene are separated from each other using a magnetic force;
3) unreacted sample and impurities are removed by washing and the sgRNA Y/dCas9-biotin complex is allowed to sequentially react with and bind to the MBs and dCas9 complex; and
4) the reaction product is allowed to react and bind with a marker capable of binding with the biotin and expression signals corresponding to at least one variation selected from optical, electrical, and chemical variations due to the catalytic action of the marker are detected.

In summary, the method uses different sgRNAs to isolate (purify) and detect the target, during which the sequences are validated twice, and prevent non-specific binding by washing between the processes to achieve improved detection performance, thus being suitable for use as sandwich assays.

The present invention will be more specifically explained with reference to the following examples. It will be evident to those skilled in the art that the scope of the present invention is not limited by these examples.

### <EXAMPLES>

The nucleotide sequences of sgRNAs used in the present invention are shown in Table 1.

**TABLE 1**

| sgRNA | Target location | SEQ ID NO: | Target recognition sequence of sgRNA and PAM |
|---|---|---|---|
| sgRNA1 | Exon1-Exon2 | 1 | AGGACATTCGTGCACCCCCA-TGG |
| sgRNA2 | Exon2-Exon3 | 2 | GAGGCCGAGAATATCACGAC-GGG |
| sgRNA3 | Exon3-Exon4 | 3 | CCTGGAAGAGGATGGAGGTC-GGG |
| sgRNA4 | Exon4-Exon5 | 4 | TCGGGCTCTGGGAGCCCAGA-AGG |

The sequences of dCas9 proteins used in the present invention are shown in Table 2.

**TABLE 2**

| SEQ ID NO: | Sequence of dCas9 protein |
|---|---|
| 5 | dCas9-3XFLAG |
| 6 | dCas9 used in dCas9-biotin |
| 7 | C80L mutant of dCas9 used in dCas9-biotin |
| 8 | C574E mutant of dCas9 used in dCas9-biotin |

### Example 1: Production and purification of dCas9 proteins

Dead Cas9 (sp. dCas9) proteins derived from *Streptococcus pyogenes* were used as dCas9s. The dCas9s were PCR-amplified from the sp. dCas9-bacteria. N-terminal 6×histidine tagged dCas9 DNA was transferred to pET28a vector. A dCas9 for subsequent biotinylation was named "dCas9H" (SEQ ID NO: 6) and a dCas9 for attachment to anti-FLAG magnetic beads was named "dCas9F" (SEQ ID NO: 5). 3 ×FLAG tag (DYKDHDGDYKDHDIDYKDDDDK) was attached to the C-terminus of the dCas9F.

The recombinant plasmid was transformed into *E. coli* BL21 (DE3) and cultured in Terrific Broth supplemented with 1 mM isopropyl β-d-1-thiogalactopyranoside (IPTG) at 18 °C for 16 h for expression. Thereafter, only the cultured bacteria were isolated by centrifugation and only the *E. coli* lysate was then isolated by ultrasonic disintegration. The *E. coli* lysate was purified using Ni-NTA resin (1 mL resin/500 mL culture solution) in a Tris buffer (50 mM Tris, 500 mM NaCl, pH 8). Washing solutions containing imidazole at linear concentration from 10 mM to 50 mM were used as Tris buffers for purification. A solution containing imidazole at 300 mM was used for elution. As a result, the purity and expression level of each of the dCas9H and the dCas9F were found to be ≥ 95% and 32 mg dCas9/L, respectively. The results of SDS-PAGE for the dCas9H and dCas9F proteins expressed and purified in *E*. *coli* are shown in Fig. 2.

### Example 2: In vitro use of exogenous human erythropoietin gene-specific sgRNAs and dCas9s

The exogenous human erythropoietin gene (SEQ ID NO: 9) was recombined into pCMV as a viral vector and used as both a substrate for reaction with the dCas9s and a model doping gene. The exogenous human erythropoietin gene consists of five exons to provide four exon-exon junctions (exon1-exon2, exon2-exon3, exon3-exon4, and exon4-exon5) (see Fig. 3). Thus, specific sgRNAs to the exon-exon junctions were synthesized. Specifically, each sgRNA was designed such that a 20 nt sequence recognition site covered the corresponding exon-exon junction and NGG PAM was directly attached thereto. One sgRNA was synthesized per exon-exon junction. The sgRNA complementary to the exon1-exon2 was named "sgRNA1", the sgRNA complementary to the exon2-exon3 was named "sgRNA2", and the sgRNA complementary to the exon3-exon4 was named "sgRNA3", and the sgRNA complementary to the exon4-exon5 was named "sgRNA4". The target recognition sequences of the sgRNAs for the exogenous human erythropoietin gene and PAMs are shown in Table 1.

Each of the sgRNAs was allowed to react with the dCas9 (dCas9H or dCas9F) in a molar ratio of 1:1 in a solution (pH 7.9) containing 50 mM Tris, 100 mM NaCl, 10 mM MgCl₂, and 1 mM dithiothreitol to form a 2 µM complex. The exogenous human erythropoietin gene (200 nM) was added to the reaction product and whether the dCas9 and the sgRNA were bound to the gene was observed for 1 h (see Figs. 4 and 5).

Fig. 4 shows the results of native-PAGE, demonstrating that the dCas9 and the sgRNAs were attached to different locations of the exogenous human erythropoietin gene and Fig. 5 shows the results of native-PAGE in Example 2, demonstrating that the dCas9 and the sgRNAs were attached to different locations of the exogenous human erythropoietin gene for different reaction times.

Referring to Figs. 4 and 5, the dCas9 was well attached to the target gene except for the case where the sgRNA No. 4 was used. A subsequent additional experiment revealed that the dCas9-sgRNA complexes were sufficiently attached to the exogenous human erythropoietin gene within a reaction time of ≤10 min.

### Example 3: Biotinylation of dCas9H

The dCas9H has two cysteine (Cys) residues that exist alone without forming disulfide bonds in the protein. Crystal structure analysis of the dCas9H revealed that the thiol groups of the cysteine residues are oriented outward toward the surface regardless of the protein structure. Thus, a strategy was used to form covalent bonds via a maleimide-thiol reaction with the cysteine residues of the dCas9H for biotinylation. To form sulfhydryl (-SH) groups at the two cysteine residues, the purified dCas9H was reduced with 100 molar equivalents of tris(2-carboxyethyl)phosphine (TCEP) at room temperature for 30 min. Then, the TCEP was removed using a 100 KDa molecular weight cut-off filter and the dCas9H was concentrated to 5 µM and 15 µM. At this time, experimental parameters were divided for optimal biotinylation. The two dCas9H concentrates were allowed to react with 10 and 20 molar equivalents of maleimide-poly(ethylene glycol)-3-biotin in a solution (pH 7.4) containing 50 mM Tris and 140 mM NaCl at 4 °C for 16 h, respectively. Thereafter, the excess biotin was removed by dialysis using a 7 KDa molecular weight cut-off filter. Finally, the biotinylated dCas9s were labeled with streptavidin-polyHRP to complete the fabrication of sensors capable of generating final detection signals.

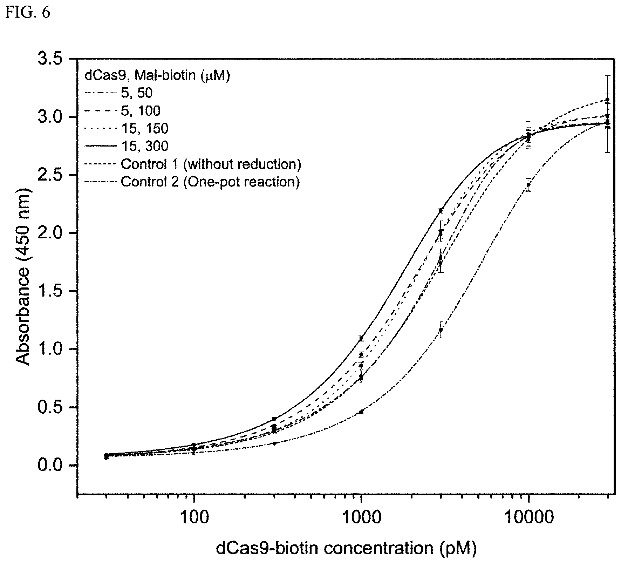

The optimization experiments for the reaction of the dCas9H protein and maleimide-poly(ethylene glycol)-3-biotin revealed that the strongest detection signals were obtained from the experimental group in which the dCas9 protein at 15 µM was treated with 20 molar equivalents of maleimide-poly(ethylene glycol)-3-biotin (see Fig. 6).

### Example 4: Abilities of dCas9H mutants with limited number of cysteine residues to detect the gene

The dCas9H has cysteine residues at positions 80 and 574 where a maximum of two biotin groups can be attached. As a result, final detection signals are mixed depending on biotinylation efficiency, causing problems in terms of signal quantification. Thus, the cysteine residues of the dCas9H were replaced with other amino acid residues by genetic engineering to establish dCas9H mutants (C80L and C574E). The abilities of the mutants to detect gene doping were compared.

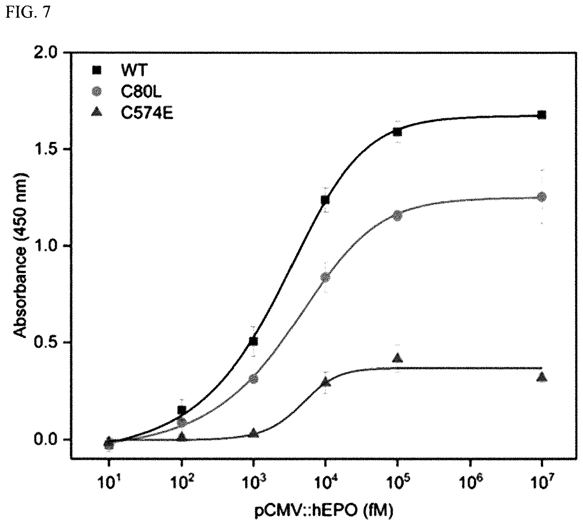

The dCas9H mutants were established by site directed mutagenesis. The exogenous gene was detected using the dCas9H mutants in the same manner as in Examples 1 and 3. The abilities of the mutants to detect the exogenous gene were investigated. First, anti-FLAG magnetic beads (MBs) were blocked with 2% bovine serum albumin (BSA) under refrigerated conditions. Thereafter, the dCas9F-sgRNA2 complex (10 nM) was immobilized onto 1 µL of the MBs in 100 µL of a reaction solution (50 mM Tris, 100 mM NaCl, 10 mM MgCl₂, pH 7.9) for 10 min. Subsequently, the complex was allowed to react with different concentrations of the exogenous human erythropoietin gene for 10 min. Each of the reaction products was washed twice with the reaction solution and 100 µL of the dCas9H-biotin (or dcas9H mutant-biotin)-sgRNA3 complex (10 nM) was added thereto. The reaction was allowed to proceed for 10 min. Thereafter, the reaction product was washed twice with the reaction solution and allowed to react with 100 µL of streptavidin-polyHRP in a dilution ratio of 1:4000 for 2 min. The reaction product was washed three times with the reaction solution and allowed to react with 90 µL of a 5,5'-tetramethylbenzidine (TMB) solution and sulfuric acid for color development. As a result, a linear quantitation range of ~10 fM to 100 nM was obtained (see Fig. 7).

The abilities of the dCas9H mutants to detect the exogenous human erythropoietin gene were judged to be satisfactory similarly to that of the wild type dCas9H. The dCas9H-C80L mutant was used for subsequent developments and experiments. As a result, a 1:1 comparison between the detection signals and the target gene was accomplished, enabling more sophisticated analysis.

### Example 5: Measurement and optimization of signal values depending on sgRNA combinations

As can be seen in Fig. 4, the three sgRNAs except for the sgRNA4 showed strong binding affinities for the exogenous human erythropoietin gene. In the previous examples, the sgRNA2 was used for the dCas9F and the sgRNA3 was used for the dCas9H-biotin. In this example, the developed exogenous gene detection method was carried out for six possible combinations of the three sgRNAs to find an optimal combination for exogenous human erythropoietin analysis.

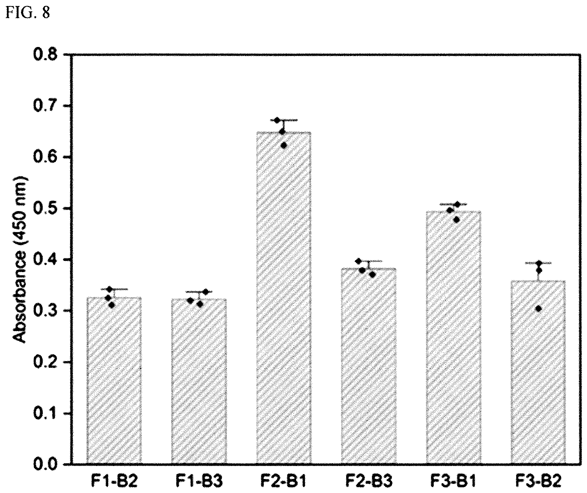

To this end, analysis was performed in the same manner as in Example 4, except that different sgRNA combinations were used for the dCas9F and the dCas9H(C80L)-biotin, the exogenous human erythropoietin gene was used at a single concentration of 100 pM, and the final TMB color development was performed for 2 min. As a result, the strongest detection signals were obtained from the dCas9F-sgRNA2 and dCas9H(C80L)-biotin-sgRNA1 combination. Therefore, it was decided to use the combination for subsequent kit developments and experiments (see Fig. 8).

### Example 6: Detection of exogenous human erythropoietin gene in whole blood

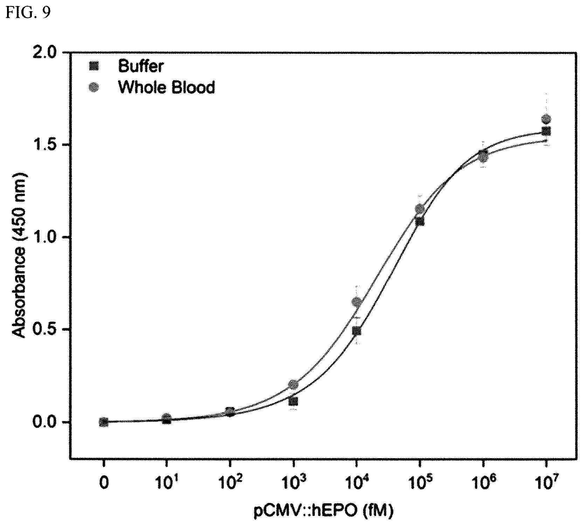

10 µL of whole blood was treated with different concentrations of the exogenous human erythropoietin gene to investigate the operation of sensors capable of rapidly identifying the exogenous human erythropoietin gene. The experimental procedure was based on that of Example 4, except that 1 U/µL of an RNase inhibitor was further used when the dCas9F and the sgRNA2 were attached to the MBs and the exogenous human erythropoietin gene was isolated. After that, the dCas9H(C80L)-biotin was allowed to react with the target in a sample. The reaction product was allowed to sufficiently react with TMB for 9 min. The final chromogenic reaction was stopped with sulfuric acid. As a result, the sensor was found to have a limit of detection (LOD) of 12.3 fM and a limit of quantification (LOQ) of 91 fM, as shown in Fig. 9. These experimental results concluded that the analytical method can differentially detect endogenous and exogenous genes from real blood samples from humans and can be utilized for gene doping analysis in practical doping applications. In addition, the direct use of the sample without blood pretreatment for analysis demonstrates the simplicity of the inventive method.

## Claims

1. A composition for identifying erythropoietin gene doping, comprising: two single guide RNAs (sgRNAs) having different nucleotide sequence set forth in any two of SEQ ID NOs: 1 to 4; and a dead Cas9 or nuclease-inactive Cas9 (dCas9) having the amino acid sequence set forth in SEQ ID NO: 5 and the dCas9 contains an affinity tag; and a dCas9 having an amino acid sequence set forth in any one of SEQ ID NOs: 6 to 8 and the dCas9 is biotinylated, and the biotin is labeled with a marker; and an expression substrate that exhibits at least one variation selected from optical, electrical, and chemical variations due to the catalytic action of the marker and comprises a chromogenic material, a luminescent material, a fluorescent material or a combination thereof.

2. The composition according to claim 1, wherein the composition comprises single guide RNA 2 (sgRNA2) having the nucleotide sequence set forth in SEQ ID NO: 2 and sgRNA3 having the nucleotide sequence set forth in SEQ ID NO: 3.

3. The composition according to claim 1, wherein the composition comprises single guide RNA 2 (sgRNA2) having the nucleotide sequence set forth in SEQ ID NO: 2 and sgRNA1 having the nucleotide sequence set forth in SEQ ID NO: 1.

4. The composition according to claim 1, wherein the affinity tag is selected from His tags, FLAG tags, S tags, glutathione S-transferase (GST) tags, maltose binding protein (MBP) tags, chitin binding protein (CMP) tags, Avi tags, calmodulin tags, polyglutamate tags, E tags, HA tags, myc tags, SBP tags, Softag 1, Softag 3, Strap tags, TC tags, Xpress tags, biotin carboxyl carrier protein (BCCP) tags, and green fluorescent protein (GFP) tags.

5. The composition according to claim 1, wherein the marker is selected from streptavidin-bound horseradish peroxidase (HRP), alkaline phosphatase (AP), β-galactosidase, luciferase, acetylcholinesterase, urease, catalase, asparginase, ribonuclease, malate dehydrogenase, staphylococcal nuclease, triose phosphate isomerase, glucose oxidase, cytochrome P450, and peroxidase compounds.

6. A kit for identifying erythropoietin gene doping, comprising: two sgRNAs having different nucleotide sequences set forth in any two of SEQ ID NOs: 1 to 4; a dCas9 labeled with an affinity tag; a biotinylated dCas9; a marker binding with the biotin; and an expression substrate that exhibits at least one variation selected from optical, electrical, and chemical variations due to the catalytic action of the marker and comprises a chromogenic material, a luminescent material, a fluorescent material or a combination thereof.

7. An information provision system for identifying erythropoietin gene doping, comprising: a targeting unit comprising a biological sample isolated from an individual suspected of gene doping, a dCas9 labeled with an affinity tag, a dCas9 labeled with a marker, and two sgRNAs having different nucleotide sequences set forth in any two of SEQ ID NOs: 1 to 4; an expression preparation unit providing an expression substrate that exhibits at least one variation selected from optical, electrical, and chemical variations due to the catalytic action of the marker bound to the dCas9 and comprises a chromogenic material, a luminescent material, a fluorescent material or a combination thereof; and an expression measurement unit determining whether the individual has gene doped or not based on the variation exhibited by the expression substrate.

8. A method for identifying erythropoietin gene doping, comprising (1) reacting a dCas9 containing an affinity tag with a sgRNA having a nucleotide sequence set forth in any one of SEQ ID NOs: 1 to 4 to form a complex, (2) immobilizing the complex onto magnetic beads via the affinity tag binding to the magnetic beads, (3) reacting the complex immobilized onto the magnetic beads with a biological sample isolated from an individual suspected of gene doping, (4) reacting the reaction product with a sgRNA having a nucleotide sequence set forth in any one of the other SEQ ID NOs: 1 to 4 and a biotinylated dCas9, (5) reacting the reaction product with a marker capable of binding with the biotin, (6) reacting the reaction product with an expression substrate that exhibits at least one variation selected from optical, electrical, and chemical variations due to the catalytic action of the marker, and (7) determining whether the individual has gene doped or not based on the variation exhibited by the expression substrate.

## Patentansprüche

1. Zusammensetzung zur Erkennung von Erythropoetin-Gendoping, umfassend: zwei Single-Guide-RNAs (sgRNAs) mit voneinander verschiedenen Nukleotidsequenzen nach zwei der SEQ IDs Nr. 1 bis 4; und eine tote Cas9 oder Nuklease-inaktive Cas9 (dCas9) mit der Aminosäuresequenz nach SEQ ID Nr. 5, wobei die dCas9 einen Affinitäts-Tag enthält; und eine dCas9 mit einer Aminosäuresequenz nach einer der SEQ IDs Nr. 6 bis 8, wobei die dCas9 biotinyliert ist und das Biotin mit einem Marker markiert ist; und ein Expressionssubstrat, das eine optische und/oder elektrische und/oder chemische Variation aufgrund der katalytischen Wirkung des Markers aufweist und ein chromogenes Material, ein lumineszierendes Material, ein fluoreszierendes Material oder eine Kombination aus diesen umfasst.

2. Zusammensetzung nach Anspruch 1, wobei die Zusammensetzung Single-Guide-RNA 2 (sgRNA2) mit der Nukleotidsequenz nach SEQ ID Nr. 2 und sgRNA3 mit der Nukleotidsequenz nach SEQ ID Nr. 3 umfasst.

3. Zusammensetzung nach Anspruch 1, wobei die Zusammensetzung Single-Guide-RNA 2 (sgRNA2) mit der Nukleotidsequenz nach SEQ ID Nr. 2 und sgRNA1 mit der Nukleotidsequenz nach SEQ ID Nr. 1 umfasst.

4. Zusammensetzung nach Anspruch 1, wobei der Affinitäts-Tag aus His-Tags, FLAG-Tags, S-Tags, Glutathion-S-Transferase-Tags (GST-Tags), Maltosebindendes-Protein-Tags (MBP-Tags), Chitin-bindendes-Protein-Tags (CMP-Tags), Avi-Tags, Calmodulin-Tags, Polyglutamat-Tags, E-Tags, HA-Tags, myc-Tags, SBP-Tags, Softag 1, Softag 3, Strap-Tags, TC-Tags, Xpress-Tags, Biotin-Carboxyl-Carrier-Protein-Tags (BCCP-Tags) und Grün-fluoreszierendes-Protein-Tags (GFP-Tags) ausgewählt ist.

5. Zusammensetzung nach Anspruch 1, wobei der Marker aus Streptavidingebundener Meerrettichperoxidase (HRP), alkaliner Phosphatase (AP), β-Galactosidase, Luciferase, Acetylcholinesterase, Urease, Katalase, Asparginase, Ribonuklease, Malatdehydrogenase, Staphylokokkennuklease, Triosephosphatisomerase, Glucoseoxidase, Cytochrom P450 und Peroxidaseverbindungen ausgewählt ist.

6. Kit zur Erkennung von Erythropoetin-Gendoping, umfassend: zwei sgRNAs mit voneinander verschiedenen Nukleotidsequenzen nach zwei der SEQ IDs Nr. 1 bis 4; eine mit einem Affinitäts-Tag markierte dCas9; eine biotinylierte dCas9; einen an das Biotin bindenden Marker; und ein Expressionssubstrat, das eine optische und/oder elektrische und/oder chemische Variation aufgrund der katalytischen Wirkung des Markers aufweist und ein chromogenes Material, ein lumineszierendes Material, ein fluoreszierendes Material oder eine Kombination aus diesen umfasst.

7. Informationsbereitstellungssystem zur Erkennung von Erythropoetin-Gendoping, umfassend: eine Targeting-Einheit, umfassend eine einem des Gendopings verdächtigten Individuum entnommene biologische Probe, eine mit einem Affinitäts-Tag markierte dCas9, eine mit einem Marker markierte dCas9 und zwei sgRNAs mit voneinander verschiedenen Nukleotidsequenzen nach zwei der SEQ IDs Nr. 1 bis 4; eine Expressionsvorbereitungseinheit, die ein Expressionssubstrat bereitstellt, das eine optische und/oder elektrische und/oder chemische Variation aufgrund der katalytischen Wirkung des an die dCas9 gebundenen Markers aufweist und ein chromogenes Material, ein lumineszierendes Material, ein fluoreszierendes Material oder eine Kombination aus diesen umfasst; und eine Expressionsmesseinheit, die anhand der Variation des Expressionssubstrats feststellt, ob das Individuum Gendoping betrieben hat oder nicht.

8. Verfahren zur Erkennung von Erythropoetin-Gendoping, umfassend (1) Reagieren einer einen Affinitäts-Tag enthaltenden dCas9 mit einer sgRNA mit einer Nukleotidsequenz nach einer der SEQ IDs Nr. 1 bis 4 zur Bildung eines Komplexes, (2) Immobilisieren des Komplexes auf Magnetic Beads mittels des an die Magnetic Beads bindenden Affinitäts-Tags, (3) Reagieren des auf den Magnetic Beads immobilisierten Komplexes mit einer einem des Gendopings verdächtigten Individuum entnommenen biologischen Probe, (4) Reagieren des Reaktionsprodukts mit einer sgRNA mit einer Nukleotidsequenz nach einer der verbleibenden SEQ IDs Nr. 1 bis 4 und einer biotinylierten dCas9, (5) Reagieren des Reaktionsprodukts mit einem Marker, der in der Lage ist, an das Biotin zu binden, (6) Reagieren des Reaktionsprodukts mit einem Expressionssubstrat, das eine optische und/oder elektrische und/oder chemische Variation aufgrund der katalytischen Wirkung des Markers aufweist, und (7) Feststellen anhand der Variation des Expressionssubstrats, ob das Individuum Gendoping betrieben hat.

## Revendications

1. Composition pour identifier le dopage génétique d'érythropoïétine, la composition comprenant : deux ARN guides simple brin (ARNsg) ayant des séquences nucléotidiques différentes selon deux quelconques des SEQ ID NOs : 1 à 4 ; et une Cas9 morte ou une Cas9 inactive à la nucléase (dCas9) ayant la séquence d'acides aminés selon la SEQ ID NO : 5 et la dCas9 contient une étiquette d'affinité ; et une dCas9 ayant une séquence d'acides aminés selon l'une quelconque des SEQ ID NOs : 6 à 8 et la dCas9 est biotinylée et la biotine est marquée avec un marqueur ; et un substrat d'expression qui présente au moins une variation sélectionnée parmi des variations optiques, électriques et chimiques à cause de l'action catalytique du marqueur et comprend une matière chromogène, une matière luminescente, une matière fluorescente ou une combinaison de celles-ci.

2. Composition selon la revendication 1, dans laquelle la composition comprend l'ARN guide simple brin 2 (ARNsg2) ayant la séquence nucléotidique selon la SEQ ID NO : 2 et l'ARNsg3 ayant la séquence nucléotidique selon la SEQ ID NO : 3.

3. Composition selon la revendication 1, dans laquelle la composition comprend l'ARN guide simple brin 2 (ARNsg2) ayant la séquence nucléotidique selon la SEQ ID NO : 2 et l'ARNsg1 ayant la séquence nucléotidique selon la SEQ ID NO : 1.

4. Composition selon la revendication 1, dans laquelle l'étiquette d'affinité est sélectionnée parmi les étiquettes His, les étiquettes FLAG, les étiquettes S, les étiquettes glutathione S-transférase (GST), les étiquettes protéine liant le maltose (MBP),les étiquettes protéine liant la chitine (CMP), les étiquettes Avi, les étiquettes calmoduline, les étiquettes polyglutamate, les étiquettes E, les étiquettes HA, les étiquettes myc, les étiquettes SBP, le Softag 1, le Softag 3, les étiquettes Strap, les étiquettes TC, les étiquettes Xpress, les étiquettes protéine porteuse de biotin carboxyl (BCCP) et les étiquettes protéine fluorescente verte (GFP).

5. Composition selon la revendication 1, dans laquelle le marqueur est sélectionné parmi la peroxydase de raifort liée à la streptavidine (HRP), le phosphatase alcaline (AP), la β-galactosidase, la luciférase, l'acétylcholinestérase, l'uréase, la catalase, l'asparginase, la ribonuclease, la malate déshydrogénase, la nucléase staphylococ-cique, la triose phosphate isomérase, la glucose oxydase, le cytochrome P450 et des composés de peroxydase.

6. Kit pour identifier le dopage génétique d'érythropoïétine, le kit comprenant : deux ARNsg ayant des séquences nucléotidiques différentes selon deux quelconques des SEQ ID NOs : 1 à 4 ; une dCas9 marquée avec une étiquette d'affinité ; une dCas9 biotinylée ; un marqueur liant à la biotine ; et un substrat d'expression qui présente au moins une variation sélectionnée parmi des variations optiques, électriques et chimiques à cause de l'action catalytique du marqueur et comprend une matière chromogène, une matière luminescente, une matière fluorescente ou une combinaison de celles-ci.

7. Système de fourniture d'information pour identifier le dopage génétique d'érythropoïétine, le système comprenant : une unité de ciblage comprenant un échantillon biologique isolé d'un individu soupçonné du dopage génétique, une dCas9 marquée avec une étiquette d'affinité, une dCas9 marquée avec un marqueur et deux ARNsg ayant des séquences nucléotidiques différentes selon deux quelconques des SEQ ID NOs : 1 à 4 ; une unité de préparation d'expression fournissant un substrat d'expression qui présente au moins une variation sélectionnée parmi des variations optiques, électriques et chimiques à cause de l'action catalytique du marqueur lié à la dCas9 et comprend une matière chromogène, une matière luminescente, une matière fluorescente ou une combinaison de celles-ci ; et une unité de mesure d'expression déterminant si l'individu s'est dopé génétiquement ou non sur la base de la variation présentée par le substrat d'expression.

8. Procédé pour identifier le dopage génétique d'érythropoïétine, le procédé comprenant les étapes consistant à (1) réagir une dCas9 contenant une étiquette d'affinité avec un ARNsg ayant une séquence nucléotidique selon l'une quelconque des SEQ ID NOs : 1 à 4 afin de former un complexe, (2) immobiliser le complexe sur des billes magnétiques par l'étiquette d'affinité liant aux billes magnétiques, (3) réagir le complexe immobilisé sur les billes magnétiques avec un échantillon biologique isolé d'un individu soupçonné du dopage génétique, (4) réagir le produit de la réaction avec un ARNsg ayant une séquence nucléotidique selon l'une quelconque des autres SEQ ID NOs : 1 à 4 et une dCas9 biotinylée, (5) réagir le produit de la réaction avec un marqueur apte à lier à la biotine, (6) réagir le produit de la réaction avec un substrat d'expression qui présente au moins une variation sélectionnée parmi des variations optiques, électriques et chimiques à cause de l'action catalytique du marqueur et (7) déterminer si l'individu s'est dopé génétiquement ou non sur la base de la variation présentée par le substrat d'expression.
